# EUROPÄISCHE PATENTANMELDUNG

(11) **EP 2 919 005 A1**
(43) Veröffentlichungstag der Anmeldung: **16.09.2015**
(21) Anmeldenummer: 15000148.5
(22) Anmeldetag: 20.01.2015
(51) Int. Cl.: G01N 31/12, G01N 1/40, G01N 33/00

(54) **VERFAHREN UND VORRICHTUNG ZUR ANALYSE VON STICKSTOFF (N) IN EINER PROBE**

(30) Priorität: 20.02.2014 DE 102014002266
(71) Anmelder: Elementar Analysensysteme GmbH, 63452 Hanau (DE)
(72) Erfinder: Federherr, Eugen, 63456 Hanau (DE); Kupka, Hans-Joachim, 63543 Neuberg (DE); Lange, Lutz, 60599 Frankfurt am Main (DE); Sieper, Hans-Peter, 63571 Gelnhausen (DE)
(74) Vertreter: Grimm, Ekkehard

(57) **Zusammenfassung**

Die Erfindung betrifft ein Verfahren zur Analyse von Stickstoff in einer Probe, wobei die Probe in einem Oxidationsreaktor unter Sauerstoffzufuhr verbrannt wird und die bei der Verbrennung entstehenden Verbrennungsgase in einem Trägergasstrom aus dem Oxidationsreaktor abgeführt werden, anschließend in diesem Gasgemisch vorhandene Stickoxide zu molekularem Stickstoff reduziert werden, dann aus dem Gasgemisch gasförmige Matrixbestandteile abgetrennt werden, so dass das verbleibende Trägergas und der darin enthaltene molekulare Stickstoff einem Detektor zur Bestimmung des Stickstoffs zugeführt werden; der in dem Trägergas enthaftene molekulare Stickstoff wird vor Zuführung zu dem Detektor aufkonzentriert. Weiterhin wird eine entsprechende Vorrichtung angegeben.

## Beschreibung

Die vorliegende Erfindung betrifft ein Verfahren zur Analyse von Stickstoff (N) in einer Probe, wobei die Probe in einem Oxidationsreaktor unter Sauerstoffzufuhr (O₂) verbrannt wird und die bei der Verbrennung entstehenden Verbrennungsgase in einem Trägergasstrom (He) aus dem Oxidationsreaktor abgeführt werden, anschließend In diesem Gasgemisch vorhandene Stickoxide (NOₓ) zu molekularem Stickstoff (N₂) reduziert werden, dann aus dem Gasgemisch gasförmige Matrixbestandteile abgetrennt werden, so dass das verbleibende Trägergas (He) und der darin enthaltene molekulare Stickstoff (N₂) einem Detektor zur Bestimmung des Stickstoffs (N₂) zugeführt werden.

Weiterhin betrifft die Erfindung eine Vorrichtung zur Analyse von Stickstoff (N) in einer Probe mit einem die Probe während der Verbrennung aufnehmenden Oxidationsreaktor, mit einer Sauerstoffzuführung zum Zuführen von Sauerstoff (O₂) in den Oxidationsreaktor und mit einer Trägergaszuführung zum Zuführen eines Trägergases (He) in den Oxidationsreaktor, wobei, in Strömungsrichtung des Trägergases gesehen, der Oxidationsreaktor mit einem Reduktionsrohr verbunden ist, dieses Reduktionsrohr mit einer Trennvorrichtung zum Abtrennen von gasförmigen Matrixbestandteilen aus dem Gasstrom verbunden ist und die Trennvorrichtung mit einem Detektor zur Bestimmung des Stickstoffs (N₂) verbunden ist.

Ein solches Verfahren sowie eine Vorrichtung mit den vorstehend angegebenen Merkmalen sind allgemein bekannt und werden auf dem Gebiet der Elementaranalyse eingesetzt Soweit vorstehend und in der nachfolgenden Beschreibung der Begriff gasförmige Matrixbestandteile verwendet wird, so werden hierunter alle Bestandteile des Gasgemischs, die neben dem Analyt in dem Gasgemisch vorhanden sind, verstanden, wobei in dem vorliegenden Verfahren der Gesamtstickstoff TN (Total Nitrogen) der Analyt ist.

Bei dem bekannten Verfahren und der bekannten Vorrichtung besteht die Problematik, dass die niedrigen Stickstoff-Konzentrationen, insbesondere im Bereich der Stabilen Isotopen Analytik, nicht oder nicht mit zureichender Genauigkeit analysiert werden können.

Die DE 35 38 778 A1 beschreibt ein Verfahren und eine Vorrichtung zum Bestimmen des Gehalts an Stickstoff in einer Probe. Die Probe wird unter Sauerstoff in einem Verbrennungsrohr (Verbrennungszone) verbrannt und die gebildeten, gasförmigen Produkte, wie Stickstoff und/oder Stickoxide, werden in eine Analyseeinrichtung überführt, in der sie als Stickstoff quantitativ nachgewiesen werden. Die Probe wird in einem mit Sauerstoff angereicherten, stickstofffreien, inerten Trägergas verbrannt und nach der Verbrennung werden in Strömungsrichtung des Trägergases gesehen vor der Verbrennungszone eventuell an der Wand des Verbrenrungsrohrs abgeschiedene Kondensationsprodukte durch Ausheizen verflüchtigt und der Analyseeinrichtung zugeführt.

Die DE 197 08 179 A1 beschreibt ein Reduktionsrohr zur Stickstoffbestimmung bei der Elementaranalyse, wobei in dem Rohr ein Metall in granulierter Form eingefüllt ist, das Stickoxide zu Stickstoff reduziert und Sauerstoff absorbiert. Als Metall wird Wolfram und/oder Molybdän eingesetzt, wobei das granulierte Material in dem Rohr, in Richtung der Rohrachse gesehen, geschichtet ist mit jeweils einer Ausdehnungspuffer-Zwischenzone, beispielsweise aus Quarzwolle.

Die DE 20 25 390 A beschreibt ein Verfahren zur quantitativen Bestimmung der in den einzelnen chemischen Verbindungen eines Stoffgemischs enthaltenen Elemente bzw. Isotopen. Das Stoffgemisch wird durch eine gaschromatografische Säule in die einzelnen Verbindungen getrennt, und diese werden dann einer chemischen Umsetzung unterworfen und detektiert. Die bei der chemischen Umsetzung entstandenen Reaktionsprodukte werden unter Vermeidung einer Vermischung in eine zweite gaschromatografische Säule eingeleitet und nach beendeter Trennung des ursprünglichen Stoffgemischs aus dieser bei erhöhter Temperatur wieder eluiert und den Detektoren zugeführt.

Demzufolge liegt der vorliegenden Erfindung die Aufgabe zugrunde, ein Verfahren und eine Vorrichtung zu schaffen, die die vorstehend beschriebenen Nachteile des Stands der Technik beseitigen bzw. eine niedrigere Bestimmungsgrenze und eine höhere Genauigkeit im unteren Könzentrationsbereich ermöglichen.

Ein Gelöst wird diese Aufgabe verfahrensgemaß durch ein Verfahren mit den Merkmalen des Anspruchs 1 und vorrichtungsgemäß durch eine Vorrichtung mit den Merkmalen des Anspruchs 9. Bevorzugte Ausführungsformen sowohl des Verfahrens als auch der Vorrichtung ergeben sich aus den abhängigen Ansprüchen.

Ein wesentliches Merkmal des erfindungsgemäßen Verfahrens ist darin zu sehen, dass der in dem Trägergas (He) enthaltende molekulare Stickstoff (N₂) vor Zuführung zu dem Detektor aufkonzentriert wird. Dadurch wird erreicht, dass die Analysenpeaks schärfer werden, was bei der gleichen Konzentration zu einem höheren Peak und somit zu einem besseren Signal-Rausch Verhältnis führt. Die Bestimmungsgrenze wird somit niedriger und die Genauigkeit im unteren Konzentrationsbereich besser. Entsprechend der Vorrichtung wird für diese Aufkonzentrierung eine Aufkonzentrierungseinrichtung eingesetzt.

Es ist vorgesehen, dass die Aufkonzentrierung durch Adsorption des molekularen Stickstoffs (N₂) an einem Adsorbermaterial erfolgt Nach vollständiger Adsorption wird der adsorbierte Stickstoff zur Zuführung zu dem Detektor von dem Adsorbermaterial desorbiert. Bevorzugt wird als Adsorbermaterial ein solches mit einer Zeolith-Struktur eingesetzt. Beispiele für dieses bevorzugte Adsorbermaterial sind Typ A, X oder Y Zeolithe. Ein Adsorbermaterial mit einer Zeolith-Struktur ermöglicht bei niedrigenen Temperaturen eine Adsorption des molekularen Stickstoffs (N₂).

Um die notwendige Retentionszeit, bei gleichzeitig minimaler Peakverbreiterung, zu erreichen, sollte das Adsorbermaterial während der Adsorption aktiv gekühlt werden. Um einen den Peak fokussierenden Effekt zu erreichen ist mit einer hohen Heizrate während der Desorption aktiv zu heizen. Die Kühlung erfolgt bei Temperaturen bis zu -30 °C und die Beheizung erfolgt bei Temperaturen bis zu 100 °C. Die Zelten, die für das Kühlen und Beheizen benötigt werden, hängen von der Menge und der Transportgeschwindigkeit (Strömungsgeschwindigkeit des Trägergases und Adsorptionsverhalten im System) des Analyts sowie dem Totvolumen des Systems ab und liegen im Bereich von ca. 2,5 Minuten für das Kühlen und im Bereich von unter einer Minute für das Beheizen.

Es hat sich als besonders bevorzugt herausgestellt, für die Kühlung und, unter Umständen auch die Beheizung, des Adsorbermaterials zur Adsorption und Desorption des molekularen Stickstoffs (N₂) den Peltier-Effekt unter Verwendung von mindestens eines entsprechenden Peltier-Elements zu nutzen. Dies hat den Vorteil, dass für das Kühlen keine Kühlmittelversorgung, wie beispielsweise flüssiger Stickstoff erforderlich ist. Unter den Umständen, dass auch das Heizen mittels der Peltier Elemente erfolgt, stellt der nicht Gebrauch von weiteren Technologien, wie z.B. Widerstandheizer, einen weiteren Vorteil da.

Das enindungsgemaße Verfahren ebenso wie die erfindungsgemäße Vorrichtung werden insbesondere auch zur Analyse von Stickstoff (TN) in einer wässrigen Probe eingesetzt, um Aufschluss über den Anteil des gesamtgebundenen Stickstoffs (TNb - Total Nitrogen bounded) In der Probe in seinen unterschiedlichen Bindungsformen ohne den Anteil von physikalisch gelöstem molekularem Stickstoff (N₂) zu erhalten. Hierzu wird in der wässrigen Probe vor der Verbrennung physikalisch gelöster molekularer Stickstoff (N₂) von dem gesamtgebundenen Stickstoff (TNb) getrennt. Nach der Trennung wird nur die Probe mit dem darin enthaltenen gesamtgebundenen Stickstoff (TNb) der Verbrennung unterworfen.

Die Trennung von physikalisch gelöstem molekularem Stickstoff (N₂) von dem gesamtgebundenen Stickstoff (TNb) erfolgt vorrichtungsgemäß vorzugsweise durch eine Vakuum-Membran-Trennvorrichtung.

Je nach Anforderung, die an die Analyse der wässrigen Probe gestellt wird, kann der abgetrennte physikalisch gelöste Stickstoff (N₂) in einem zusätzlichen Verfahrensschritt detektiert werden. Eine solche Detektion des in der Probe vorhandenen physikalisch gelösten, molekularen Stickstoff (N₂) kann somit in einem Verfahrensablauf neben der Detektierung des in dem gesamtgebundenen Stickstoffs (TNb) enthaltenen molekularen Stickstoffs (N₂) erfolgen.-Die Trennung ist dann von Bedeutung, wenn nicht nur der Gesamtstickstoff (TN) von Interesse ist, sondern auch der darin enthaltene, gebundene Stickstoff (TNb) und/oder der physikalisch gelöster Stickstoff. Voraussetzung für das Letztere ist, dass die Konzentration oder stabile Isotopen Zusammensetzung des physikalisch gelösten Stickstoffs eine Aussagekraft besitzt. Dies ist abhängig von dem Untersuchungsobjekt (Probe) und dem Zweck der Untersuchung.

In dem vorstehend beschriebenen Verfahrensablauf wird, unabhängig davon, ob abgetrennter physikalisch gelöster oder aus TNb nach erfolgter Verbrennung und Reduktion erstandener, molekularer N₂ vor Zuführung zu dem Detektor einer Aufreinigung unterworfen wird, um störende Matrixbestandteile (Wasser, Halogenverbindungen und Kohlendioxid) abzutrennen. Diese Bestandteile (Wasser, Halogenverbindungen) können ansonsten zur Korrosion und Beschädigung von empfindlichen Bauteilen und im Bereich der Stabilen Isotopen Analytik zu falschen Ergebnissen durch isobare Interferenzen (Wasser, Kohlendioxid) und zur Minderung der Adsorptionskapazität/-effizienz des Stickstoff-Adsorbers (Wasser, Kohlendioxid) führen.

Eine Abtrennung von Wasser erfolgt vorrichtungsgemäß durch einen Kondensor, einen Membrantrockner und einen chemischen Absorber, eine Abtrennung von Halogenverbindungen durch einen Halogen-Absorber und eine Abtrennung von Kohlendioxid durch einen CO₂-Ab- oder Adsorber.

Weitere Einzelheiten und Merkmale der Erfindung ergeben sich aus der nachfolgenden Beschreibung von Ausführungsbeispielen anhand der Zeichnung. In der Zeichnung zeigt
- Figur 1: einen Verfahrensablauf zur Analyse von Stickstoff in einer Probe gemäß einer ersten Ausführungsform,
- Figur 2: einen Verfahrensablauf zur Analyse von Stickstoff in einer Probe gemäß einer zweiten Ausführungsform, der die Verfahrensschritte des in Figur 1 dargestellten Verfahrens umfasst, jedoch für die Analyse von Stickstoff in einer wässrigen Probe eingesetzt wird, um nur die Probe mit dem darin enthaltenen gesamtgebundenen Stickstoff (TNb) der Verbrennung in einem Oxidationsreaktor zuzuführen,
- Figur 3: eine Vorrichtung gemäß der Erfindung, mit der das in Figur 1 dargestellte Verfahren durchführbar ist, und
- Figur 4: eine Vorrichtung gemäß der Erfindung, mit der das in Figur 2 dargestellte Verfahren durchführbar ist.

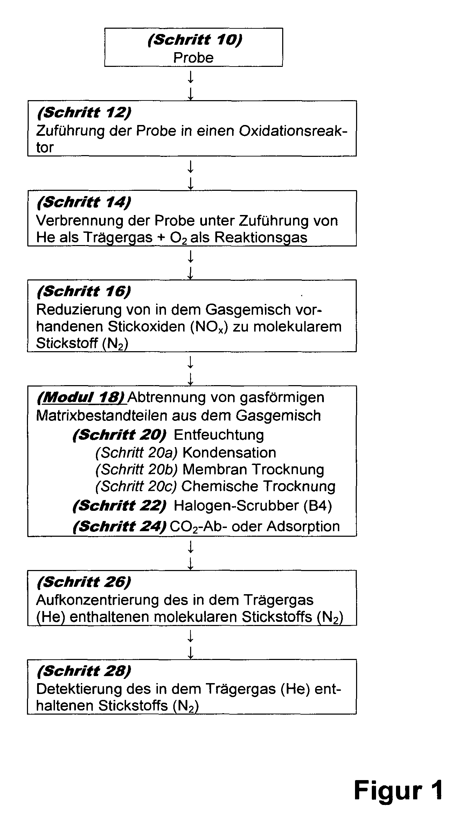

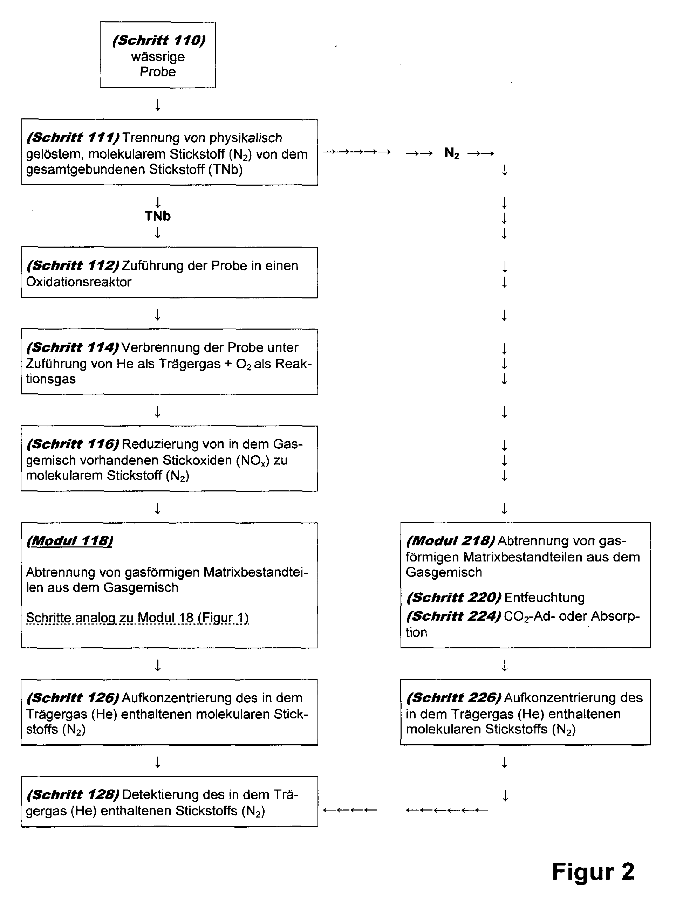

Die beiden Verfahrensabläufe, wie in den Figuren 1 und 2 dargestellt sind, sind zur Analyse von Stickstoff (N) in einer Probe vorgesehen.

Unter Bezugnahme zunächst auf Figur 1 wird in einem Verfahrensschritt 10 eine auf ihren Gehalt an Stickstoff (N) zu analysierende Probe bereitgestellt. Diese Probe wird in einem nächsten Schritt 12 einem Oxidationsreaktor zugeführt und anschließend, Schritt 14, unter Zuführung von Helium (He) als Trägergas und Sauerstoff (O₂) als Reaktionsgas verbrannt. Die Verbrennungsgase werden in dem Trägergasstrom aus dem Oxidationsreaktor abgeführt, und im Schritt 16 werden die in diesem Gasgemisch vorhandenen Stickoxide (NOₓ) zu molekularem Stickstoff (N₂) reduziert. Um interferenzen, Korrosionsschäden sowie Kapazitäts- und Effizienzminderung des Adsorbers auszuschließen, werden dann gasförmige Matrixbestandteile aus dem Gasgemisch abgetrennt (Schritt 18). Bei den gasförmigen Matrixbestandteilen handelt es sich um alle gasförmigen Bestandteile ohne den Analyt, also dem Gesamtstickstoff (TN).

Weiterhin wird der Gasstrom in einem Schritt 20 entfeuchtet, In einem Schritt 22 werden die Halogene entfernt und in einem Schritt 24 erfolgt eine CO₂-Ad- oder Absorption.

Während die dargestellte Abfolge der Schritte 20 bis 24 bevorzugt ist, da dadurch zum Beispiel das adsorbierte CO₂ zur Kohlenstoff-Analyse verwendet werden kann, kann die Abfolge der Schritte 20 bis 24 auch geändert werden, indem Schritt 22 vor einem der Teilschritte des Schritts 20, nämlich die Membrantrocknung (Teilschritt 20b), vorgenommen wird. Die angegebene Reihenfolge der Schritte 20a, 22, 20b, 20c und 24 hat den Vorteil, dass die für die Trocknung verwendete Membraneinheit (Schritt 20b) vor aggressiven Halogenspezies geschützt wird.

Die aufgereinigte Gasmischung könnte dann unmittelbar dem Verfahrensschritt 28 unterworfen werden, indem der in dem Trägergas enthaltene molekulare Stickstoff (N₂) detektiert wird. Gemäß einem besonderen Aspekt der Erfindung wird jedoch vor der Detektion im Schritt 28 ein Verfahrensschritt 26 durchgeführt, in dem der in dem Trägergas (He) enthaltene molekulare Stickstoff (N₂) aufkonzentriert wird. Durch diese Aufkonzentrierung wird eine symmetrische Form (kein Tailing, Splitting usw.) und eine bessere Fokussierung des Peaks erreicht, und dadurch wird ein besseres Signal/Rausch Verhältnis erzielt. Durch diese Verstärkung des Signals bei der Detektion des molekularen Stickstoffs (N₂) in dem Verfahrensschritt 28 derart, dass sich das Signal während der Detektion von dem Hintergrundsignal absetzt, können auch geringe Mengen an in dem Trägergas (He) enthaltenem, molekularen Stickstoff (N₂) mit hoher Genauigkeit detektiert werden.

Bei der Analyse des Gehalts an Stickstoff (N) in einer wässrigen Probe kann es von Interesse sein, welcher Anteil an Gesamtstickstoff (TN) bzw. welcher Anteil der Zusammensetzung der stabilen Isotopen auf den physikalisch gelösten, molekularen Stickstoff (N₂) oder den gesamtgebundenen Stickstoff (TNb) zurückgeht. Um hierüber Aufschluss zu erhalten, wird eine zu analysierende, wässrige Probe (siehe Schritt 110 in Figur 2) zunächst einer Trennung von physikalisch gelöstem, molekularem Stickstoff (N₂) von dem gesamtgebundenen Stickstoff (TNb) aus Gesamtstickstoff (TN) unterworfen (Schritt 111).

Wie ein Vergleich des in Figur 2 dargestellten Verfahrensablaufs mit demjenigen Verfahrensablauf, der in Figur 1 dargestellt und zuvor beschrieben ist, zeigt, sind Verfahrensschritte des Verfahrensablaufs der Figur 2, die den Verfahrensschritten der Figur 1 entsprechen, mit denselben Bezugszeichen, ergänzt um 100 oder 200, bezeichnet. Insofern können die Erläuterungen zu den jeweiligen Verfahrensschritten der Figur 1 auf die entsprechenden Verfahrensschritte der Figur 2, oder vice versa, übertragen werden.

Der in dem Trennschritt 111 verbleibende gesamtgebundene Stickstoff (TNb) wird den bereits anhand der Figur 1 erläuterten Verfahrensschritten 112 bis 128 (Schritte 12 bis 28 in Figur 1) unterzogen. Allerdings geht der in den Verfahrensablauf im Schritt 128 detektierte molekulare Stickstoff (N₂) nur auf den molekularen Stickstoff (N₂) zurück, der in dem im Schritt 111 verbleibenden gesamtgebundenen Stickstoff (TNb) in gebundener Form vorliegt.

Falls nun zusätzlich auch der Anteil an molekularem Stickstoff (N₂), der in der wässrigen Probe in physikalisch gelöster Form vorlag, quantitativ erfasst werden soll, wird der in dem Verfahrensschritt 111 aus der Probe getrennte Anteil des gelösten molekularen Stickstoffs (N₂) den Verfahrenschritten 220,224 und 226 unterzogen. In dem Verfahrensschritt-Modul 218 (bestehend aus Verfahrensschrift 220 und 224) werden gasförmige Matrixbestandteile aus dem Gasgemisch abgetrennt bzw. entfernt, das Gasgemisch wird entfeuchtet (Schritt 220) und einer CO₂-Ad- oder Absorption (Schritt 224) unterworfen. Der Schritt 226 dient wiederum dazu, den in dem Trägergas (He) enthaltenen molekularen Stickstoff (N₂) vor der Detektion (Schritt 128) aufzukonzentrieren.

Anhand der Figur 2 ist zu erkennen, dass die Detektion der beiden Stickstoff-Anteile nach der jeweiligen Aufkonzentrierung in den Schritten 126 und 226 durch ein und dieselbe Detektionseinrichtung (Schritt 128) nacheinander erfolgen kann, was natürlich von Vorteil, jedoch nicht zwingend erforderlich ist.

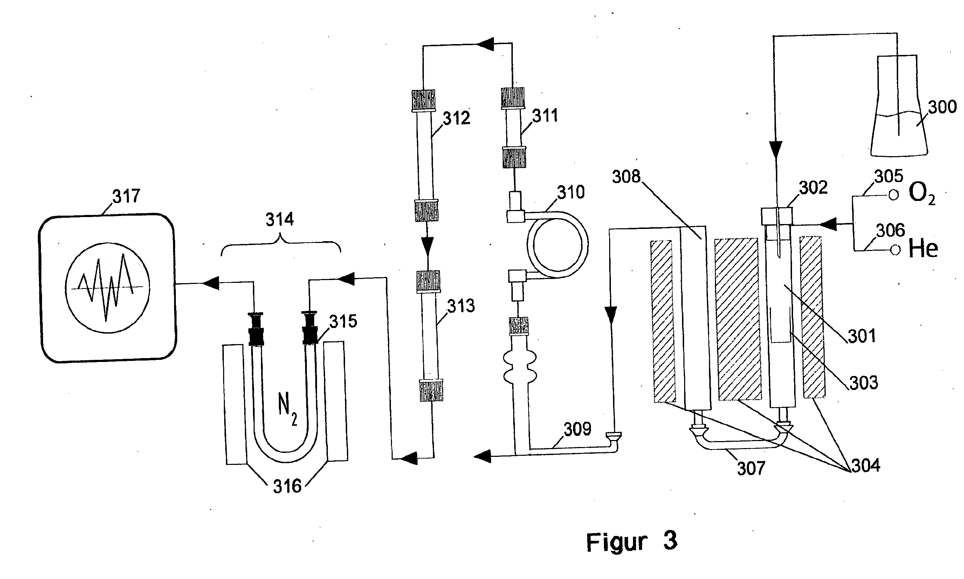

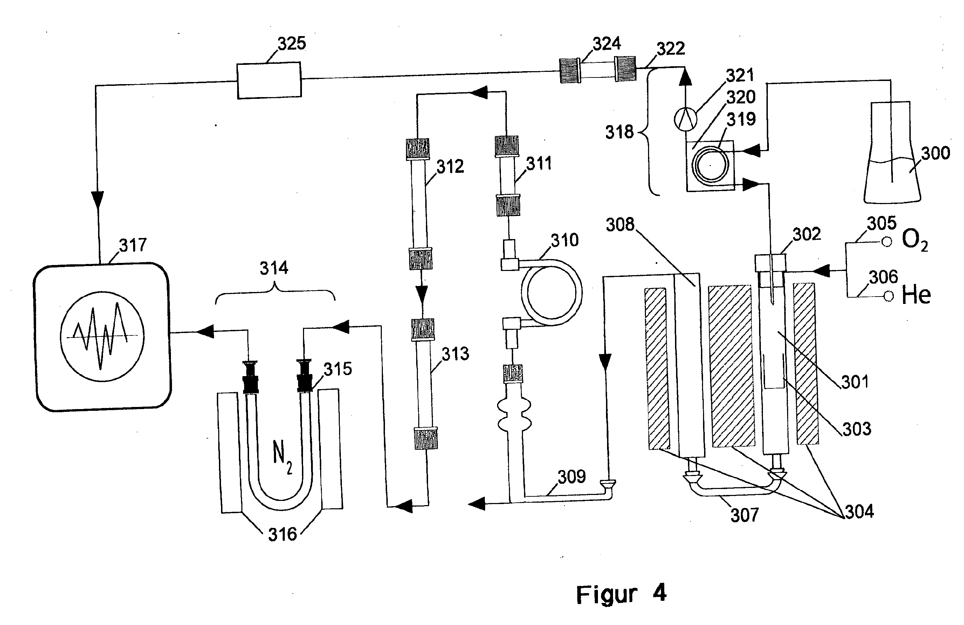

Das Verfahren, wie es in Figur 1 dargestellt ist, kann mit einer Vorrichtung der Figur 3 durchgeführt werden. Diese Vorrichtung der Figur 3 ist in der Figur 4 durch eine Trennvorrichtung erweitert, die dem Verfahrensschritt 111 des Verfahrensablaufs der Figur 2 zuzuordnen ist.

Die Vorrichtung, wie sie in Figur 3 dargestellt ist, wird zur Analyse von Stickstoff (N) in einer Probe eingesetzt

Die zu analysierende Probe (eine wässrige Probe) wird einem Probenbehälter 300 entnommen und an der Oberseite eines vertikal ausgerichteten Oxidationsreaktor 301 über einen Eingabekopf 302 in einen Probentiegel 303 injiziert. Der Oxidationsreaktor 301 ist durch einen Ofen 304 von der Außenseite aus auf Temperaturen bis zu 1200 °C (typische Verbrennungstemperatur 850 °C) beheizbar.

Die Probe wird unter Zuführung von Sauerstoff (O₂) über eine Sauerstoffzuführung 305 verbrannt und die Verbrennungsbestandteile werden unter Zuführung eines Trägergases (Helium (He)) durch eine Trägergaszuführung 306, die beide mit dem Eingabekopf 302 verbunden sind, abgeführt. Am unteren Ende ist der Oxidationsreaktor 301 über eine beheizte Gasleitung 307 mit einem ebenfalls von außen beheizten, vertikalen Reduktionsrohr 308 verbunden. In diesem Reduktionsrohr 308 werden in dem Gasgemisch (Trägergasstrom mit Restsauerstoff, sowie Analyt und Matrixbestandteile) enthaltene Stickoxide (NOₓ) bei Temperaturen von bis zu 850 °C (typischerweise 500 °C) zu molekularem Stickstoff (N₂) reduziert.

Der Trägergasstrom wird dann einer Trennvorrichtung 309 in Form eines Kondensators zugeführt, in dem der größte Anteil an Wasser aus dem Gasstrom abgetrennt wird. Der Trennvorrichtung 309 folgt eine weitere Trocknungseinrichtung 310, um den Wassergehalt (Feuchte) im Gasstrom weiter zu reduzieren. Als Trocknungseinrichtung 310 wird bevorzugt eine Membrantrocknung verwendet, die den Vorteil hat, dass diese regenerierbar ist. Anschließend wird der Gasstrom einer weiteren Trocknungseinrichtung 311 in Form eines chemischen Trockners zugeführt. Bei der chemischen Trocknung wird die Restfeuchte chemisch gebunden. Der chemische Trockner 311 enthält hierzu Phosphorpentoxid auf einem Trägermaterial. Ein weiterer Vorteil des chemischen Trockners 311 ist die Funktion als visueller Indikator, um die Funktionalität aller drei Funktionseinheiten 309, 310 und 311 (Wasserentfernung) zu überwachen. Dies wird durch eine beigemengte Indikatorsubstanz ermöglicht.

In Richtung des Gasstroms gesehen (die Strömungsrichtung ist durch die Pfeile angedeutet) führt dann der Gasstrom durch einen Halogen-Absorber 312 und eine COₓ-Adsorber- oder Absorptionseinrichtung 313. Der Halogen-Absorber 312 enthält einen Absorber (z.B. Silberwolle), so dass die Halogenverbindungen, wie z.B. HCl, chemisch gebunden werden. Die sich daran anschließende CO₂-Ad- oder Absorptionseinrichtung 313 ad- oder absorbiert Kohlenstoffdioxid (CO₂), der in dem Gasstrom enthalten ist; hierzu ist die CO₂-Ad- oder Absorptionseinrichtung 313 mit Silicagel (Adsorption) oder NaOH auf einem Träger (Absorption) gefüllt.

Der nun noch in dem Gasstrom enthaltene molekulare Stickstoff (N₂) wird in einer Aufkonzentrierungseinheit 314 aufkonzentriert. Diese Aufkonzentrierungseinheit 314 umfasst ein Adsorptionsrohr 315 (optional mit einem Mantel-Widerstandsheizer umwickelt), das von außen durch ein oder mehrere Peltier-Element(e) 316 gekühlt oder auch beheizt wird. Für den Schritt der Aufkonzentrierung wird das Adsorptionsrohr 315 zuerst aktiv gekühlt, so dass der Analyte (N₂) an dem Adsorber adsorbiert wird. Nach einer ausreichenden Adsorptionszeit wird das Adsorptionsrohr 315 aktiv, und zwar wahlweise nur durch mindestens ein Peltier-Element 316, zur Desorption besetzt, so dass dann der aufkonzentrierte, in dem Trägergas (He) enthaltene molekulare Stickstoff (N₂) einem Detektor 317 zugeführt wird.

Anstelle einer Beheizung des Adsorptionsrohrs 315 der Aufkonzentrierungseinheit zur Desorption des aufkonzentrierten Stickstoffs (N₂) mittels Peltier-Element 316 könnte auch eine nicht dargestellte Widerstandsheizung eingesetzt werden. Das heizen mittels Peltier-Element 316 hat allerdings den Vorteil, dass sowohl Kühlung als auch Aufheizung mit ein und demselben Element vorgenommen werden können. Außerdem kann zum Aufheizen des Adsorptionsrohrs 315 eine Widerstandsheizung zusätzlich zu dem Peltier-Element 316 eingesetzt werden.

Da der in dem Trägergas enthaltene molekulare Stickstoff (N₂) vor der Detektion mit dem Detektor 317 aufkonzentriert wird, wird in dem Detektor 317 ein Detektionssignal erhalten, das ein besseres Signal/Rausch Verhältnis aufweist.

In der Figur 4, die nachfolgend beschrieben wird, sind alle Teile, die mit der Vorrichtung der Figur 3 identisch sind, mit denselben Bezugszeichen bezeichnet, so dass eine erneute Beschreibung und Erläuterung dieser Teile nachfolgend weitgehendst nicht vorgenommen wird. In Bezug auf diese Teile kann auf die vorstehende Beschreibung der Figur 3 zurückgegriffen werden.

Um das Verfahren durchzuführen, das anhand des Verfahrensablaufs der Figur 2 dargestellt ist, wird in der Vorrichtung der Figur 4 eine wässrige Probe, bei der der molekulare Stickstoff (N₂), der dem Gesamtstickstoff (TN) zuzuordnen ist, von dem in der Probe vorhandenen gesamtgebundenen Stickstoff (TNb) unterschieden werden soll, zunächst einer Trennvorrichtung 318 zugeführt. Bevorzugt handelt es sich dabei um eine sogenannte Vakuum-Membran-Trennvorrichtung. In dieser Trennvorrichtung 318 führt die dem Probenbehälter 300 entnommene wässrige Probe durch einen dünnwandigen Membranschlauch 319, der durch ein gegenüber Atmosphärendruck auf Unterdruck befindliches Gehäuse 320 verläuft. Durch den Membranschlauch 319 diffundiert der physikalisch gelöste molekulare Stickstoff (N₂) in das Gehäuse 320 und wird aus dem Gehäuse 320 über eine Pumpe 321 abgesaugt. Der Probenstrom mit dem verbleibenden Stickstoff (TNb) wird dann von der Trennvorrichtung 318 bzw. aus dem Membranschlauch 319 dem Oxidationsreaktor 301 zugeführt, wie dies den Verfahrensschritten 111 und 112 der Figur 2 entspricht, und letztendlich dem Detektor 317 zugeführt, so dass in dem Detektor 317 derjenige Anteil an in dem Trägergas enthaltenen molekularen Stickstoff (N₂) analysiert wird, der nicht in der Probe als physikalisch gelöster molekularer Stickstoff (N₂) vorliegt.

Falls nun quantitative Informationen über den in der Probe enthaltenen physikalisch gelösten molekularen Stickstoff (N₂) erforderlich sind, wird der aus der Trennvorrichtung 318 über die Pumpe 321 und die Gasleitung 322 abgezogene physikalisch gelöste molekulare Stickstoff (N₂) über ein Aufreinigungs-Modul 324 geführt, das symbolisch für eine Einheit zur Abtrennung von gasförmige Matrixbestandteile aus dem Gasgemisch (Modul 218), bestehend aus einer Entfeuchtungseinrichtung (Schritt 220) und einer CO₂-Adsorber- oder Absorptionseinrichtung (Schritt 224) steht. Hierbei sind die Entfeuchtungseinrichtung mit der Trocknungseinrichtung 311 (optional in Kombination mit 310) und die CO₂-Ad- oder Absorptionseinrichtung mit der Einrichtung 313 vergleichbar.

Schließlich wird der in dem Tragergasstrom enthaltene molekulare Stickstoff (N₂) in einer weiteren Aufkonzentrierungseinheit 325 aufkonzentriert, und dann wird der aufkonzentrierte Stickstoff (N₂) durch Desorption dem Detektor 317 zugeführt, der dann nur den Anteil des in der Probe enthaltenen physikalisch gelösten molekularen Stickstoffs (N₂) bestimmt. Die weitere Aufkonzentrierungseinheit 325 entspricht vorzugsweise in ihrem Aufbau der Aufkonzentrierungseinheit 314, die zuvor beschrieben wurde.

## Patentansprüche

1. Verfahren zur Analyse von Stickstoff (N) in einer Probe, wobei die Probe in einem Oxidationsreaktor (112) unter Sauerstoffzufuhr (O₂) verbrannt wird (114) und die bei der Verbrennung (114) entstehenden Verbrennungsgase in einem Trägergasstrom (He) aus dem Oxidationsreaktor (112) abgeführt werden, anschließend in diesem Gasgemisch vorhandene Stickoxide (NOₓ) zu molekularem Stickstoff (N₂) reduziert werden (116), dann aus dem Gasgemisch gasförmige Matrixbestandteile abgetrennt werden (118), so dass das verbleibende Trägergas (He) und der darin enthaltene molekulare Stickstoff (N₂) einem Detektor zur Bestimmung des Stickstoffs (N₂) zugeführt werden (128), **dadurch gekennzeichnet, dass** der in dem Trägergas (He) enthaltene molekulare Stickstoff (N₂) vor Zuführung zu dem Detektor aufkonzentriert wird (126).

2. Verfahren nach Anspruch 1, **dadurch gekennzeichnet, dass** die Aufkonzentrierung (126) des molekularen Stickstoffs (N₂) durch Adsorption an einem Adsorbermaterial erfolgt und vor Zuführung zu dem Detektor von dem Adsorbermaterial desörbiert wird.

3. Verfahren nach Anspruch 2, **dadurch gekennzeichnet, dass** als Adsorbermaterlal ein solches mit einer Zeolith-Struktur eingesetzt wird.

4. Verfahren nach Anspruch 2 oder 3, **dadurch gekennzeichnet, dass** das Adsorbermaterial während der Adsorption aktiv gekühlt wird und während der Desorption aktiv beheizt wird.

5. Verfahren nach einem der Ansprüche 1 bis 4, **dadurch gekennzeichnet, dass** zur Analyse von Stickstoff (TN) in einer wässrigen Probe vor der Verbrennung (114) physikalisch gelöster molekularer Stickstoff (N₂) von dem gesamtgebundenen Stickstoff (TNb) getrennt wird (111) und nur die Probe mit dem darin enthaltenen gesamtgebundenen Stickstoff (TNb) der Verbrennung (114) unterworfen wird.

6. Verfahren nach Anspruch 5, **dadurch gekennzeichnet, dass** der abgetrennte physikalisch gelöste Stickstoff (N₂) nach einer Aufreinigung (218) dem Detektor zugeführt wird.

7. Verfahren nach Anspruch 6, **dadurch gekennzeichnet, dass** vor der Detektierung (128) eine Aufkonzentrierung (226) des molekularen Stickstoffs (N₂) erfolgt.

8. Vorrichtung zur Analyse von Stickstoff (N) in einer Probe mit einem die Probe während der Verbrennung aufnehmenden Oxidationsreaktor (301), mit einer Sauerstoffzuführung (305) zum Zuführen von Sauerstoff (O₂) in den Oxidationsreaktor (301) und mit einer Trägergaszuführung (306) zum Zuführen eines Trägergases (He) in den Oxidationsreaktor (301), wobei, in Strömungsrichtung des Trägergases gesehen, der Oxidationsreaktor (301) mit einem Reduktionsrohr (308) verbunden ist, dieses Reduktionsrohr (308) mit einer Trennvorrichtung (309, 310, 311, 312, 313) zum Abtrennen von gasförmigen Matrixbestandteilen aus dem Gasstrom verbunden ist und die Trennvorrichtung (309, 310, 311, 312, 313) mit einem Detektor (317) zur Bestimmung des Stickstoffs (N₂) verbunden ist, **dadurch gekennzeichnet, dass** in Strömungsrichtung des Trägergases (He) gesehen vor dem Detektor (317) eine in dem Trägergas (He) enthaltenen molekularen Stickstoff (N₂) aufkonzentrierende Aufkonzentrierungseinheit (314) angeordnet ist.

9. Vorrichtung nach Anspruch 8, **dadurch gekennzeichnet, dass** die Aufkonzentrierungseinheit (314) ein molekularen Stickstoff (N₂) adsorbierendes Adsorbermaterial enthält.

10. Vorrichtung nach Anspruch 9, **dadurch gekennzeichnet, dass** das Adsorbermaterial ein solches mit einer Zeolith-Struktur ist.

11. Vorrichtung nach einem der Ansprüche 8 bis10, **dadurch gekennzeichnet, dass** die Aufkonzentrierungseinheit (314) mittels einer Kühleinrichtung (316) kühlbar und mittels einer Heizeinrichtung (315) beheizbar Ist.

12. Vorrichtung nach Anspruch 11, **dadurch gekennzeichnet, dass** die Kühleinrichtung und/oder die Heizeinrichtung durch mindestens ein Peltier-Element (316) gebildet ist/sind.

13. Vorrichtung nach einem der Ansprüche 8 bis 12, **dadurch gekennzeichnet, dass** zur Analyse von Stickstoff (TN) in einer wässrigen Probe der Oxldationsreaktor (301) mit einer den physikalisch gelösten molekularen Stickstoff (N₂) von dem gesamtgebundenen Stickstoff (TNb) trennende Trennvorrichtung (318) verbunden ist.

14. Vorrichtung nach Anspruch 13, **dadurch gekennzeichnet, dass** die Trennvorrichtung (318) mit einer den abgetrennten physikalisch gelösten Stickstoff (N₂) aufreinigenden Aufreinigungseinrichtung (324) verbunden ist, die ihrerseits mit dem Detektor (317) verbunden ist.

15. Vorrichtung nach Anspruch 14, **dadurch gekennzeichnet, dass** zwischen der Aufreinigungseinrichtung (324) und dem Detektor (317) eine den molekularen Stickstoff (N₂) aufkonzentrierende weitere Aufkonzentrierungseinrichtung (325) angeordnet ist.
